(19) Europäisches Patentamt

European Patent Office

Office européen des brevets

(11) **EP 4 494 565 B1**

(12) **EUROPEAN PATENT SPECIFICATION**

(45) Date of publication and mention
of the grant of the patent:
**03.12.2025 Bulletin 2025/49**

(21) Application number: **24184355.6**

(22) Date of filing: **25.06.2024**

(51) International Patent Classification (IPC):
*A61B 6/12* (2006.01)     *A61B 6/00* (2024.01)
*G06T 7/00* (2017.01)

(52) Cooperative Patent Classification (CPC):
**A61B 6/5211; A61B 6/12; G06T 5/50;**
G06T 2207/10116; G06T 2207/30052

(54) **RADIOPAQUE OBJECT LOCATION APPARATUS AND METHOD**

VORRICHTUNG UND VERFAHREN ZUR ORTUNG EINES RÖNTGENDICHTEN OBJEKTS

APPAREIL ET PROCÉDÉ DE LOCALISATION D'OBJET RADIO-OPAQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **20.07.2023 US 202363527867 P
31.05.2024 US 202418679968**

(43) Date of publication of application:
**22.01.2025 Bulletin 2025/04**

(73) Proprietor: **Siemens Healthineers International
AG
6312 Steinhausen (CH)**

(72) Inventor: **MACEK, Kristijan
8048 Zurich (CH)**

(74) Representative: **Mathisen & Macara LLP
Charta House
30-38 Church Street
Staines-upon-Thames TW18 4EP (GB)**

(56) References cited:
**EP-B1- 1 760 658    US-A1- 2017 140 532**

- **AHMED IBRAHIM ABDULRAB ET AL: "Multi-
Techniques for Analyzing X-ray Images for Early
Detection and Differentiation of Pneumonia and
Tuberculosis Based on Hybrid Features",
DIAGNOSTICS, vol. 13, no. 4, 20 February 2023
(2023-02-20), CH, pages 814, XP093228216, ISSN:
2075-4418, DOI: 10.3390/diagnostics13040814**

**Description**

Technical Field

These teachings relate generally to treating a patient's planning target volume with energy pursuant to an energy-based treatment plan and more particularly to leveraging the presence of radiopaque objects.

Background

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Fiducials are sometimes used to help locate a given patient volume. Fiducial refers to a reference marker that is placed within the field of view of an x-ray image. Fiducials are often used to provide a spatial reference of scale within the image to aid in measurements, alignment, and/or calibration. Fiducials can be, for example, physical markers comprised of materials that are visible on x-ray images. Unfortunately, accurately locating a fiducial in a patient image can sometimes be challenging.

US Patent Publication No: 2017/140532 A1 discloses a method for detecting the location of thin metallic guide wires (which are radio-opaque) on x-ray images. European Patent No: 1 760 658 B1 discloses a method of constructing a gray value model of an anatomic entity in a digital medical image. "Multi-Techniques for Analyzing X-ray Images for Early Detection and Differentiation of Pneumonia and Tuberculosis Based on Hybrid Features", by Ahmed Ibrahim Abdulrab Et Al, DIAGNOSTICS, vol. 13, no. 4, 20 February 2023, page 814, discloses methods for identifying Pneumonia and Tuberculosis using X-ray images.

Summary of the Invention

According to a first aspect of the invention, a method is provided. The method comprising: by a control circuit: accessing information regarding at least one field of view of a particular patient, which field of view contains information regarding at least one radiopaque object; processing the information regarding the at least one field of view using a plurality of different filters to yield a plurality of filtered fields of view that are each different from one another, each filtered field of view comprising a differently filtered version of the at least one field of view; fusing the plurality of filtered fields of view, using multi-cue probabilistic fusion, to yield fused information regarding the location of the at least one radiopaque object by suppressing spurious detections.

According to another aspect of the invention, the information regarding the at least one field of view comprises an x-ray image.

According to another aspect of the invention, the information regarding the at least one field of view comprises only a single x-ray image.

According to another aspect of the invention, the at least one radiopaque object comprises a fiducial.

According to another aspect of the invention, the fiducial comprises an implanted fiducial.

According to another aspect of the invention, the plurality of different filters includes at least one image filter.

According to another aspect of the invention, each of the plurality of different filters comprises an image filter.

According to another aspect of the invention, the plurality of different filters includes at least one of: a contour detection filter; a bandpass filter; an adaptive thresholding filter; a fiducial structure enhancement filter; a maximally stable extremal regions filter; a homogeneity filter. filter; a fiducial structure enhancement filter; a maximally stable extremal regions filter; a homogeneity filter.

According to another aspect of the invention, the fused information regarding the at least one radiopaque object comprises a probabilistic-based likelihood image.

According to another aspect of the invention, the probabilistic-based likelihood image identifies a most-likely location of the at least one radiopaque object.

According to a second aspect of the invention, an apparatus is provided. The apparatus comprising: a control circuit (101) configured to: access information regarding at least one field of view of a particular patient, which field of view contains information regarding at least one radiopaque object; process the information regarding the at least one field of view using a plurality of different filters to yield a plurality of filtered fields of view that are each different from one another, each filtered field of view comprising a differently filtered version of the at least one field of view; fuse the plurality of filtered fields of view, using multi-cue probabilistic fusion, to yield fused information regarding the location of the at least one radiopaque object by suppressing spurious detections.

According to another aspect of the invention, the information regarding the at least one field of view comprises an x-ray image.

According to another aspect of the invention, the information regarding the at least one field of view comprises only a single x-ray image.

According to another aspect of the invention, the at least one radiopaque object comprises a fiducial.

According to another aspect of the invention, the fiducial comprises an implanted fiducial.

According to another aspect of the invention, the plurality of different filters includes at least one image filter.

According to another aspect of the invention, each of the plurality of different filters comprises an image filter.

According to another aspect of the invention, the plurality of different filters includes at least one of: a contour detection filter; a bandpass filter; an adaptive thresholding filter; a fiducial structure enhancement filter; a maximally stable extremal regions filter; a homogeneity filter.

According to another aspect of the invention, the fused information regarding the at least one radiopaque object comprises a probabilistic-based likelihood image.

According to another aspect of the invention, the probabilistic-based likelihood image identifies a most-likely location of the at least one radiopaque object.

Brief Description of the Drawings

The above needs are at least partially met through provision of the radiopaque object location apparatus and method described in the following detailed description, particularly when studied in conjunction with the drawings, wherein:

FIG. 1 comprises a block diagram as configured in accordance with various embodiments of these teachings;

FIG. 2 comprises a flow diagram as configured in accordance with various embodiments of these teachings;

FIG. 3 comprises an x-ray image as configured in accordance with various embodiments of these teachings;

FIG. 4 comprises a plurality of filtered images as configured in accordance with various embodiments of these teachings;

FIG. 5 comprises a plurality of images as configured in accordance with various embodiments of these teachings;

FIG. 6 comprises two images as configured in accordance with various embodiments of these teachings;

FIG. 7 comprises a graph as configured in accordance with various embodiments of the invention;

FIG. 8 comprises a graph as configured in accordance with various embodiments of these teachings;

FIG. 9 comprises a graph as configured in accordance with various embodiments of the invention;

FIG. 10 comprises an x-ray image as configured in accordance with various embodiments of these teachings;

FIG. 11 comprises a plurality of filtered images as configured in accordance with various embodiments of these teachings;

FIG. 12 comprises a plurality of images as configured in accordance with various embodiments of these teachings;

FIG. 13 comprises a plurality of images as configured in accordance with various embodiments of these teachings;

FIG. 14 comprises as graph as configured in accordance with various embodiments of these teachings;

FIG. 15 comprises a graph as configured in accordance with various embodiments of these teachings;

FIG. 16 comprises a graph as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

Detailed Description

Generally speaking, pursuant to these various embodiments a control circuit can access information regarding at least one field of view of a particular patient, which field of view contains information regarding at least one radiopaque object. The control circuit can then process that information using a plurality of different filters to yield a plurality of filtered fields of view that are each different from one another. Those filtered fields of view can then be fused to yield fused information regarding the aforementioned radiopaque object (or objects).

By one approach, the aforementioned information regarding at least one field of view comprises an X-ray vision. These teachings will, however, readily accommodate other imaging modalities.

By one approach, the aforementioned information regarding at least one field of view comprises only a single image (such as, for example, a single X-ray image). If desired, however, additional images (including images from dissimilar imaging modalities) can be accessed and processed.

By one approach, the aforementioned radiopaque object comprises a fiducial. Such a fiducial may be implanted with the particular patient, though these teachings will readily accommodate other placement methods. By another approach, in lieu of the foregoing or in combination therewith, the aforementioned radiopaque object may comprise a patient structure, such as a bone.

When the information regarding the at least one field of view comprises one or more images, the aforementioned plurality of different filters can include, as desired, one or more image filters. These teachings are highly flexible in practice and will accommodate use of any of a variety of image filters. Some examples include, but are certainly not limited to, a contour detection filter, a bandpass filter, an adaptive thresholding filter, a fiducial structure enhancement filter, a maximally stable extremal regions filter, and/or a homogeneity filter.

These teachings will also accommodate any of a variety of fusion methodologies. By one approach, the aforementioned fusion of the plurality of filtered fields yields a probabilistic-based likelihood image that identifies, for example, a most-likely location of the at least one radiopaque object.

That resultant information can then be used, for example, to facilitate accurately optimizing a radiation treatment plan for the particular patient and/or setting up a radiation treatment platform to administer a corresponding treatment plan, where the latter is based upon having accurately located the aforementioned radiopaque object(s).

So configured, these teachings can improve the robustness of target detection. This can result in particular in more robust tracking of structures in an x-ray imaging system, such as kV or MV detector platforms.

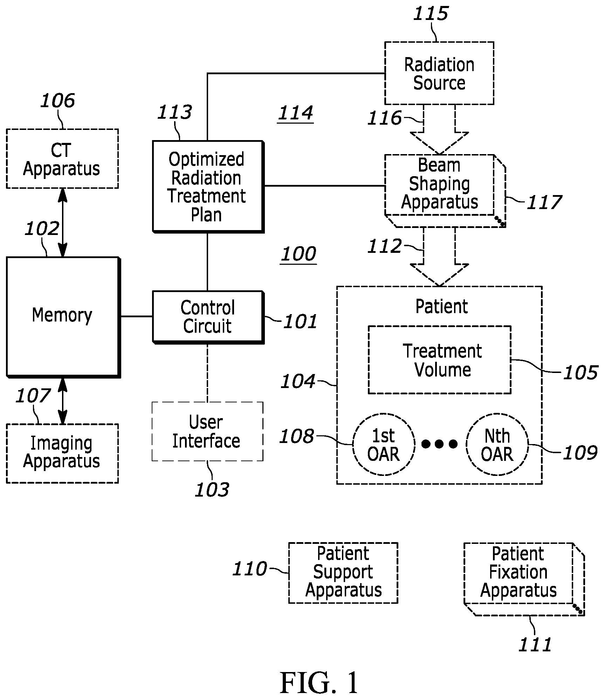

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101).

In addition to information such as optimization information for a particular patient, information regarding a particular radiation treatment platform as described herein, the information regarding one or more fields of view of a particular patient described herein, and/or the plurality of different filters as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In one illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

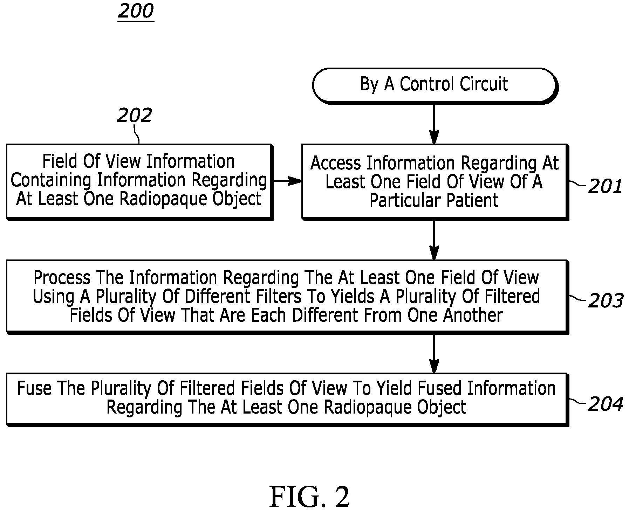

Referring now to FIG. 2, a process 200 that can be carried out, for example, in conjunction with the above-described application setting (and more particularly via the aforementioned control circuit 101) will be described. Generally speaking, this process 200 can serve to facilitate generating and/or delivering an optimized radiation treatment plan 113 to thereby facilitate treating a particular patient with therapeutic radiation using a particular radiation treatment platform per that optimized radiation treatment plan and/or properly setting up such a platform at a time of treatment to facilitate accurately administering such a plan.

At block 201, this process 200 provides for accessing information 202 regarding at least one field of view of a particular patient, which field of view contains information regarding at least one radiopaque object.

For many application settings, that information can comprise an x-ray image. These teachings will accommodate, however, images produced by other imaging modalities.

In many cases, the foregoing information 202 can comprise only a single image, such as a single x-ray image. If desired, however, these teachings will accommodate accessing a plurality of images (for example, x-ray images of a patient's area of interest from different fields of view, such as from 90 degrees apart from one another). When using a plurality of images, those images may, or may not (as desired) share a common image capture modality.

These teachings will accommodate any number of radiopaque objects, ranging from one onwards. That said, in many application settings there will be a relatively few number of fiducials, ranging from, for example, one to five or so. When there is a plurality of radiopaque objects involved, those objects may be identical to one another or at least some of the objects may be different from others of the objects as desired.

These teachings will also accommodate a variety of radiopaque objects. By one approach, for example, the radiopaque object may comprise an anatomical structure that comprises a part of the particular patient, such as a bone. By another approach, in lieu of the foregoing or in combination therewith, the radiopaque object may comprise a fiducial. The present teachings will accommodate using both implanted fiducials as well as fiducials that are not implanted.

At block 203, the control circuit 101 processes the foregoing accessed information 202 regarding the at least one field of view of the particular patient using a plurality of different filters. When the information 202 includes one or more images, one or more (including, if desired, all) of the filters may comprise an image filter. These teachings will accommodate any of a wide variety of such filters. Some useful examples include, but are not limited to:

a contour detection filter;

a bandpass filter;

an adaptive thresholding filter;

a fiducial structure enhancement filter;

a maximally stable extremal regions filter;

a homogeneity filter.

By one approach, the foregoing processing yields a plurality of filtered fields of view that are each different from one another. By way of example, when the information 202 comprises a single x-ray image depicting three fiducials, the filtered fields of view will comprise differently-filtered versions of that single image that depicts those three fiducials. In such a case, each of those images, though likely somewhat different in appearance from one another, each share the same field of view.

At block 204, the control circuit 101 then fuses the foregoing plurality of filtered fields of view to yield fused information regarding, for example, the at least one radiopaque object. By one approach, that fused information comprises a probabilistic-based likelihood image that identifies, for example, a most-likely location of the one or more radiopaque objects.

So configured, these teachings can provide a multi-cue probabilistic representation by fusing available target candidates from a number of different image processing filters into a common framework where final target positions can be more readily extracted. Multi-cue probabilistic fusion can be additive in the sense that new information from additional imaging filters can simply be integrated as new weighted evidence. Accordingly, the disclosed approach is highly flexible. In at least some cases, adding additional imaging filters/cues can help to make the overall detection estimation more robust in the sense of an increased likelihood of detecting correct target positions while also tending to suppress spurious detections (such as false positive or false negative responses).

Some more specific examples that accord with the present teachings will now be presented. It will be understood that the specific details of these examples are intended to serve an illustrative purpose and are not intended to suggest any particular limitations with respect to these teachings.

When formulating a sequential target tracking problem within a Bayesian framework where the hidden (estimated) target state is $\{x_t;\ t \in 1:T\}$ and the set of observations is $\{z_t;\ t \in 1:T\}$ (image frames), there are in general two steps that are performed recursively.

First, a prediction step

$$p(x_t|z_{1:t-1}) = \int p(x_t|x_{t-1})p(x_{t-1}|z_{1:t-1})\ dx$$

where $p(x_{t-1}|z_{1:t-1})$ is the posterior probability estimate from the previous step $t$ - 1 and a $p(x_t|x_{t-1})$ prediction (motion) model.

And second, an observation/correction step

$$p(x_t|z_{1:t}) = \frac{p(z_t|x_t)p(x_t|z_{1:t-1})}{p(z_t|z_{1:t-1})}$$

where $p(z_t|x_t)$ denotes the observation model likelihood and the $p(z_t|z_{1:t-1})$ is a normalization factor.

In the case when no motion model is available and the predicted target state in current step t is uniform (target detection only), the current posterior estimate reduces to:

$$p(x_t|z_{1:t}) = p(x_t|z_t) \propto p(z_t|x_t)$$

The state estimate can then be found as:

$$\widehat{x_t} = argmax_x\big(p(z_t|x_t)\big)$$

When multiple image filters or cues are processed, then the compounded observation model likelihood can be described as:

$$p(z_t|x_t) = \sum_{k=1}^{K} \lambda_t^k\ p\big(z_t^k|x_t\big)$$

where $\lambda_t^k$ is the weight of the kth cue with $\sum_{k=1}^{K} \lambda_t^k = 1$. Each image cue can be also assigned a constant $\lambda^k$ if there is no online adaptation.

Two examples for a multi-cue fusion approach, in particular the image processing and cue fusion to obtain the observation model $p(z_t|x_t)$, will now be presented. The two examples differ from one another with respect to patient anatomy and fiducial type, the number of image filtering cue types and fusion number thereof, and a single cue computation method.

This first example involves a patient's prostate, arbitrarily shaped fiducials, six imaging filter cues, and a single cue likelihood computation based upon a reference template cross correlation score.

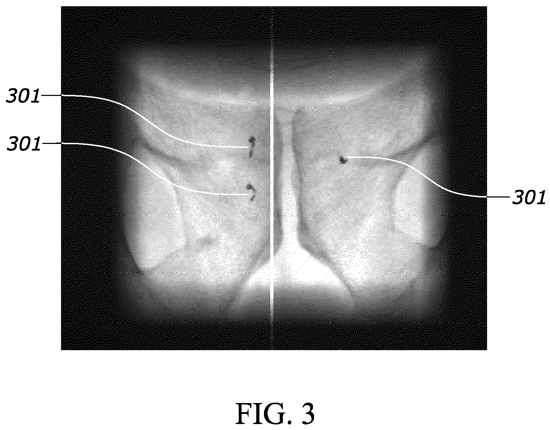

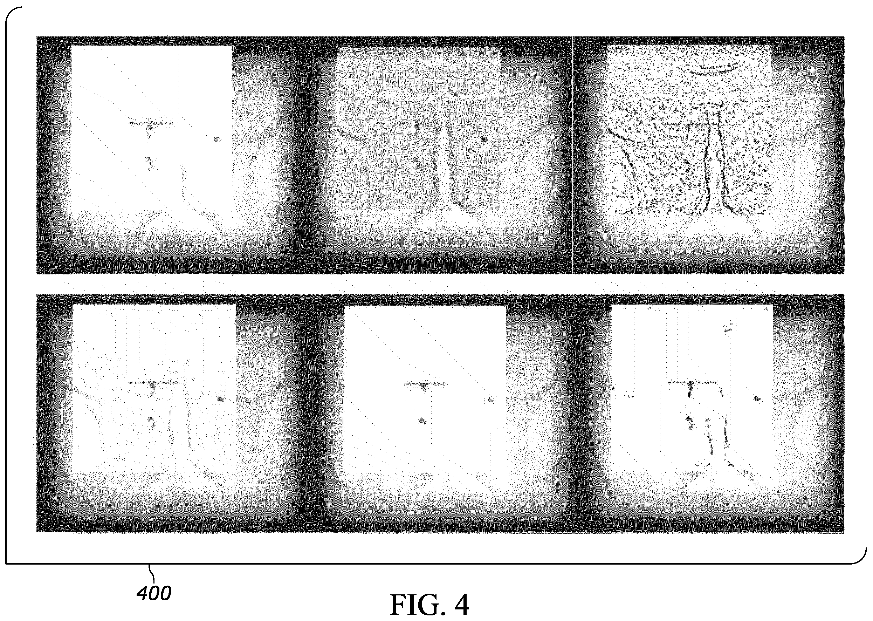

FIG. 3 presents an initial image comprising an x-ray kV projection image 300 of a pelvis use case that includes three fiducial surrogates 301. FIG. 4, in turn, presents six filtered images 400 of the foregoing image 300. The six filters employed in this example are a contour detection filter, a bandpass filter, an adaptive thresholding filter, a fiducial structure enhancement filter, a maximally stable extremal regions filter, and a homogeneity filter (from top left to bottom right).

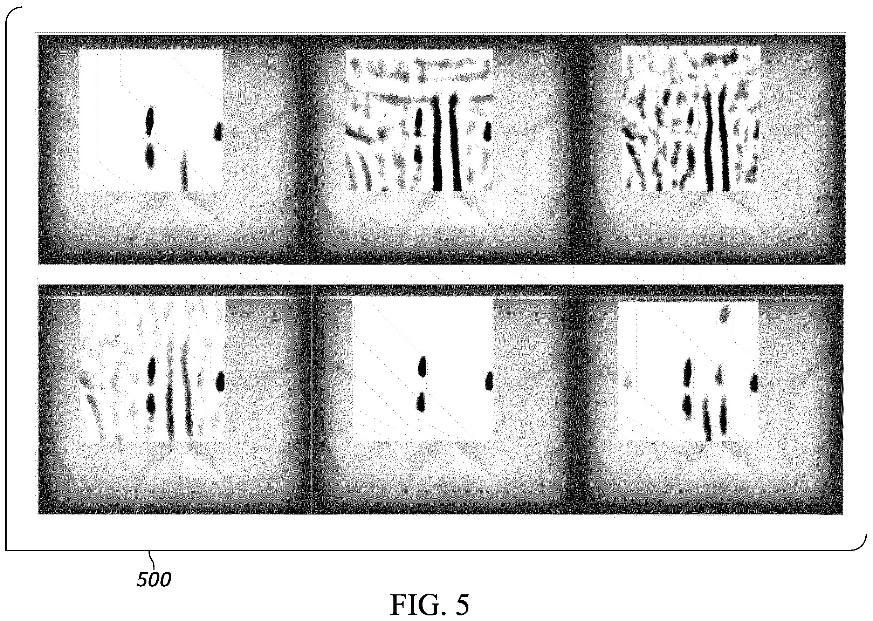

FIG. 5 presents six corresponding images 500 depicting single cue observation likelihoods based on a cross correlation score. A reference template for the top left fiducial (planning computed tomography extracted fiducial image) is cross correlated with the output corresponding to the aforementioned different imaging filters. The corresponding cross correlation score observation likelihood maps are computed for each individual fiducial separately in this example.

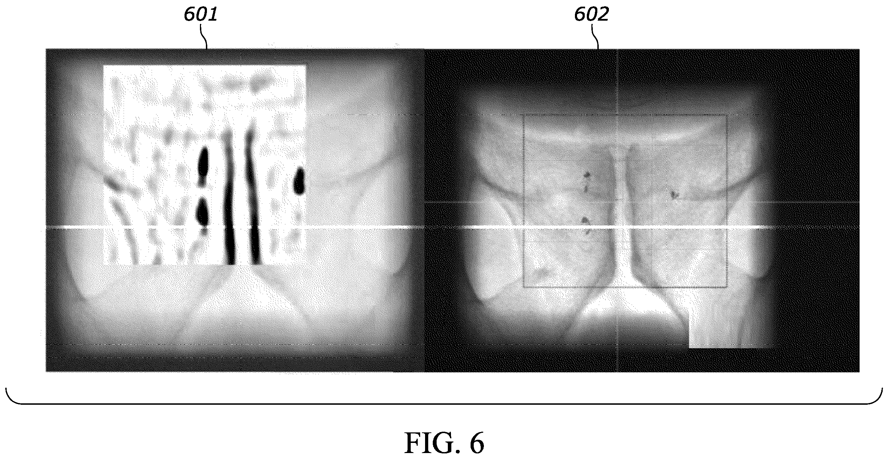

Referring now to FIG. 6, the combined multi-cue observation likelihood based on the cross correlation score is presented in the left side image 601. The likelihood is highest at the actual fiducial target location due to the individual cue voting contribution. Different image filter spurious responses along anatomy and the background are diminished since the spurious response and voting along the same false structures is less likely. An image 602 that depicts a final fiducials detection appears at the right.

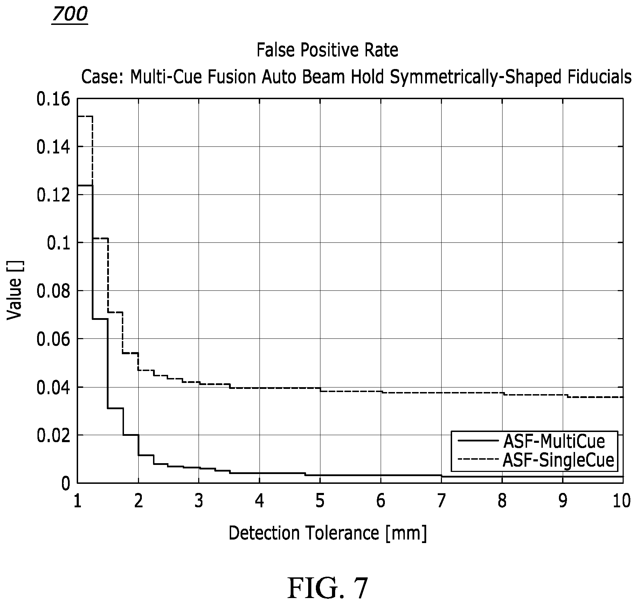

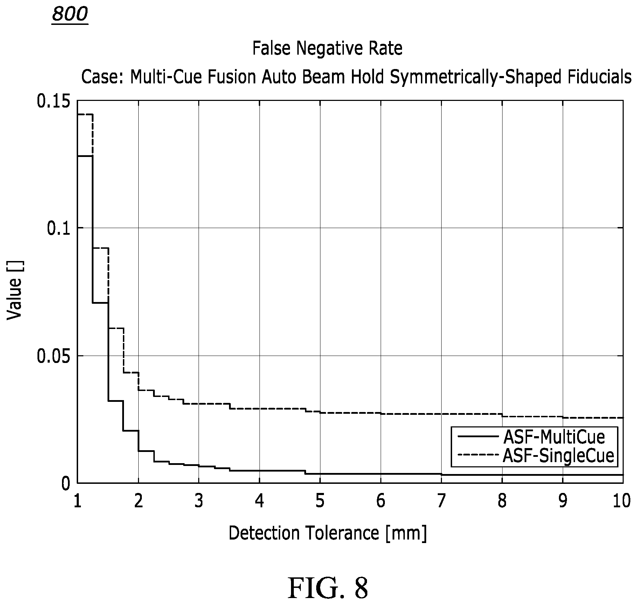

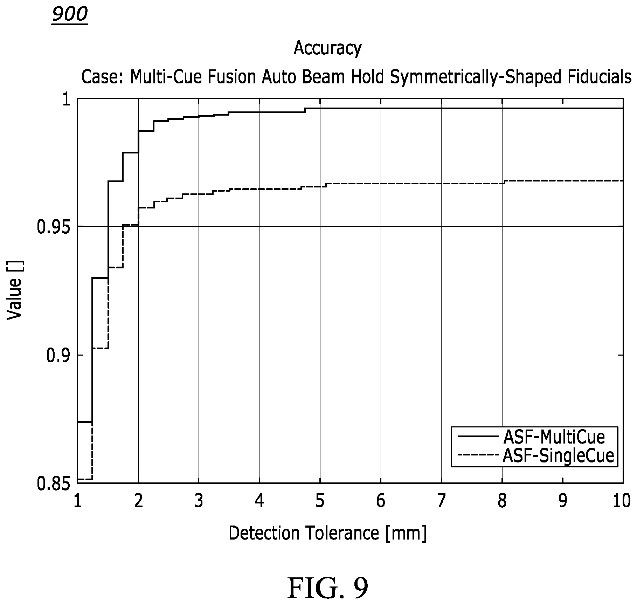

FIGS. 7-9 illustrate confusion matrix performance as corresponds to the foregoing example, with FIG. 7 presenting False-Positive Rate information 700, FIG. 8 presenting False-Negative Rate information 800, and FIG. 9 presenting Accuracy information 900 at different ground truth detection tolerances. It can be seen that overall detection performance/robustness is significantly higher when using multiple image filters/cues (full line) fusion rather than a single image filter/cue (bandpass filter) (dashed line).

This next example addresses a patient's lung, presumes the use of embolization coil fiducials, makes use of three imaging filter cues, and the single cue likelihood computation employs bounding box occupancy likelihood.

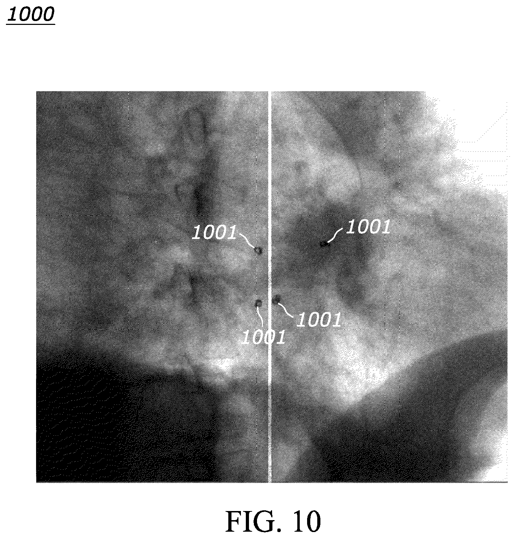

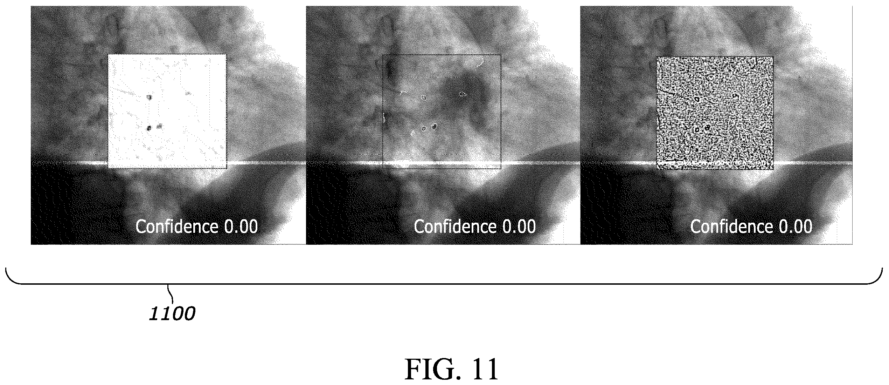

FIG. 10 presents an initial starting point x-ray kV projection image 1000 depicting the patient's lung and four fiducials 1001. FIG. 11, in turn, presents the corresponding filtered images 1100, comprising, left to right, a blobs detection filtered image, a contour detection filtered image, and a homogeneity/morphology filtered image.

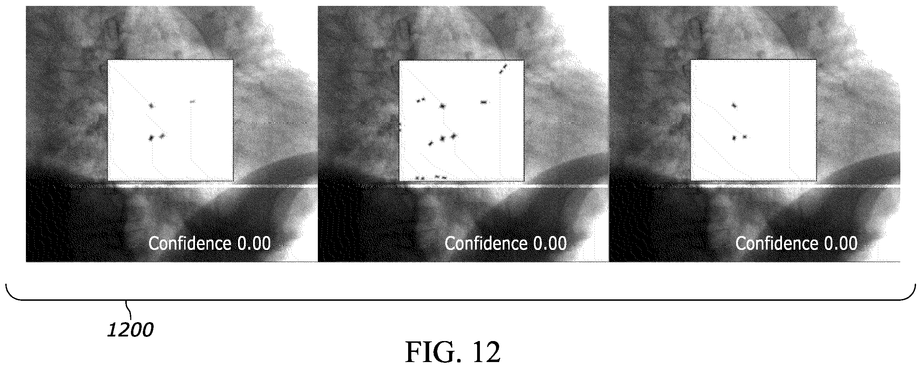

FIG. 12 presents single cue observation likelihoods images 1200 based on bounding box occupancy. At some spots there might be isolated spurious (due, for example, to contour detection in the middle) or missing candidates (see the homogeneity/morphology filter at the right) due to noisy or incorrect detection of a single image processing cue. Moreover, bounding box candidates can also be of different sizes, since they were extracted via different underlying image features.

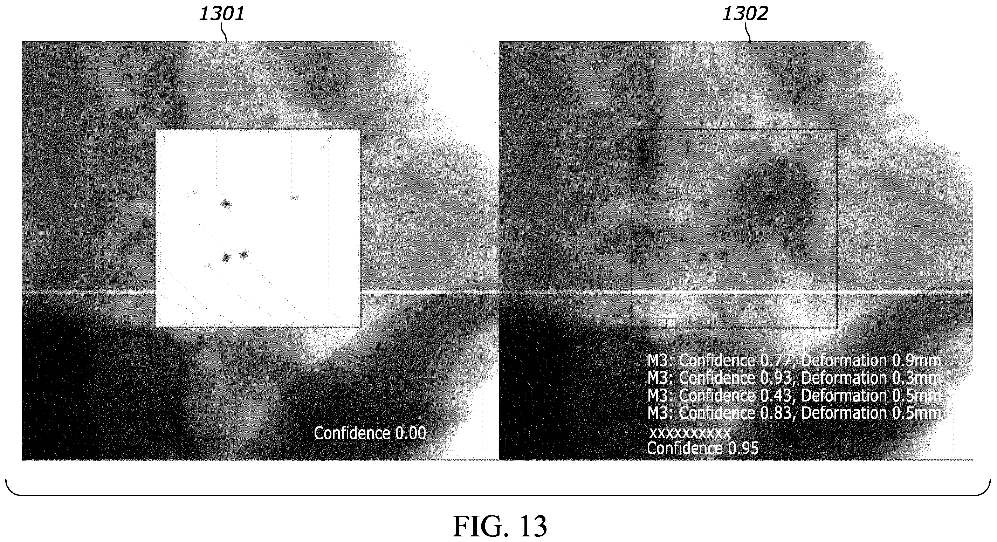

FIG. 13 presents a combined multi-cue observation likelihoods images 1301 based on bounding box occupancy. The likelihood is highest at the actual fiducial target locations due to the individual cue voting contribution. Different image filter spurious responses along anatomy and the background are diminished since the spurious response and voting along the same false structures is less likely. Final fiducials detection is presented in the image 1302 at the right.

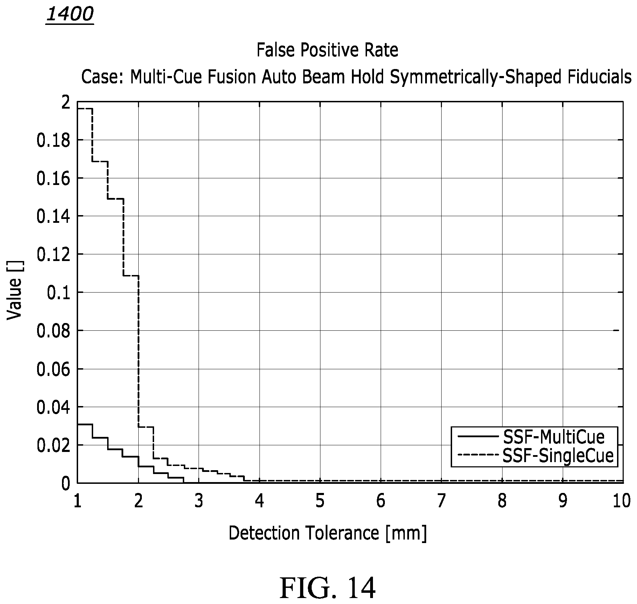

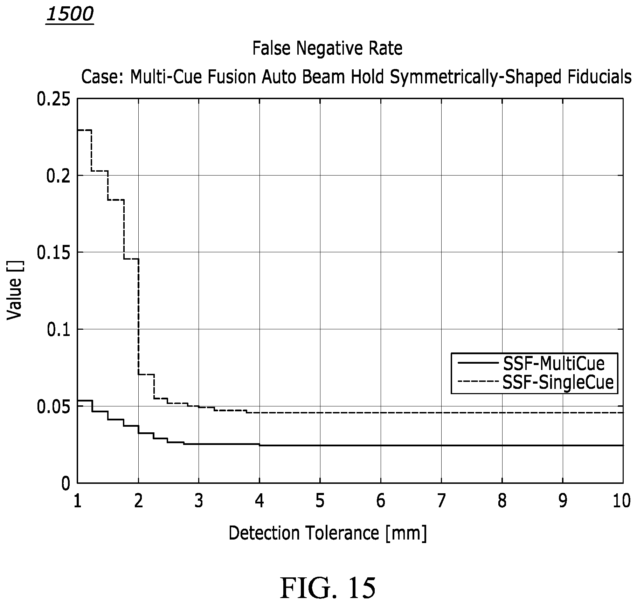

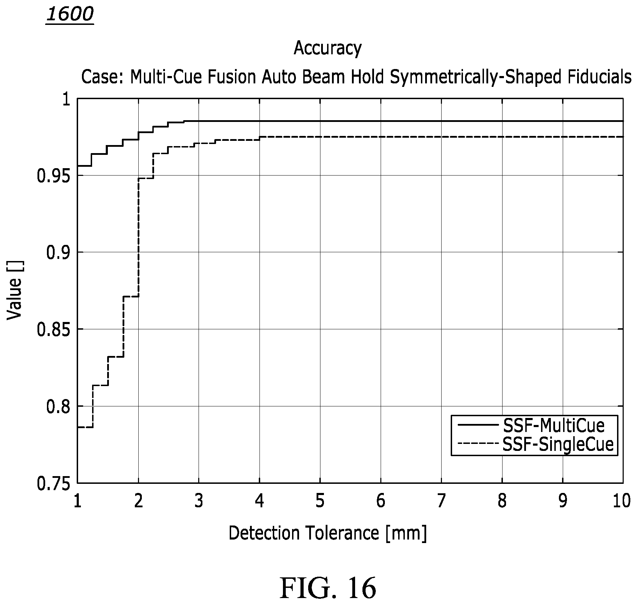

FIGS. 14-16 present information regarding confusion matrix performance as regarding the foregoing example. The graph 1400 in FIG. 14 presents False-Positive Rate performance, the graph 1500 in FIG. 15 presents False-Negative Rate performance, and the graph 1600 in FIG. 16 presents Accuracy performance at different ground truth detection tolerances. The overall detection performance/robustness is shown to be significantly higher when using multiple image filters/cues (full lines) fusion as compared to a single image filter/cue (blobs detection) (dashed lines).

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

**Claims**

1.   A method comprising:
       by a control circuit (101):

           accessing information regarding at least one field of view of a particular patient, which field of view contains information regarding at least one radiopaque object;
           processing the information regarding the at least one field of view using a plurality of different filters to yield a plurality of filtered fields of view that are each different from one another, each filtered field of view comprising a differently filtered version of the at least one field of view; and
           **characterised by**:
           fusing the plurality of filtered fields of view, using multi-cue probabilistic fusion, to yield fused information regarding the location of the at least one radiopaque object by suppressing spurious detections.

2.   The method of claim 1, wherein the information regarding the at least one field of view comprises an x-ray image; and optionally, wherein the information regarding the at least one field of view comprises only a single x-ray image.

3.   The method of claim 1 or claim 2, wherein the at least one radiopaque object comprises a fiducial; and optionally, wherein the fiducial comprises an implanted fiducial.

4.   The method of any one of claims 1 to 3, wherein the plurality of different filters includes at least one image filter.

5.   The method of claim 4, wherein each of the plurality of different filters comprises an image filter.

6.   The method of claim 4, wherein the plurality of different filters includes at least one of:

           a contour detection filter;
           a bandpass filter;
           an adaptive thresholding filter;
           a fiducial structure enhancement filter;

a maximally stable extremal regions filter;
a homogeneity filter.

7. The method of any one of claims 1 to 6, wherein the fused information regarding the at least one radiopaque object comprises a probabilistic-based likelihood image.

8. The method of claim 7, wherein the probabilistic-based likelihood image identifies a most-likely location of the at least one radiopaque object.

9. An apparatus comprising:
a control circuit (101) configured to:

access information regarding at least one field of view of a particular patient, which field of view contains information regarding at least one radiopaque object;
process the information regarding the at least one field of view using a plurality of different filters to yield a plurality of filtered fields of view that are each different from one another, each filtered field of view comprising a differently filtered version of the at least one field of view; and
**characterised by**:
fuse the plurality of filtered fields of view, using multi-cue probabilistic fusion, to yield fused information regarding the location of the at least one radiopaque object by suppressing spurious detections.

10. The apparatus of claim 9, wherein the information regarding the at least one field of view comprises an x-ray image; and optionally, wherein the information regarding the at least one field of view comprises only a single x-ray image.

11. The apparatus of claim 9 or claim 10, wherein the at least one radiopaque object comprises a fiducial; and optionally, wherein the fiducial comprises an implanted fiducial.

12. The apparatus of any one of claims 9 to 11, wherein the plurality of different filters includes at least one image filter.

13. The apparatus of claim 12, wherein each of the plurality of different filters comprises an image filter.

14. The apparatus of claim 12, wherein the plurality of different filters includes at least one of:

a contour detection filter;
a bandpass filter;
an adaptive thresholding filter;
a fiducial structure enhancement filter;
a maximally stable extremal regions filter;
a homogeneity filter.

15. The apparatus of any one of claims 9 to 14, wherein the fused information regarding the at least one radiopaque object comprises a probabilistic-based likelihood image; and optionally, wherein the probabilistic-based likelihood image identifies a most-likely location of the at least one radiopaque object.

**Patentansprüche**

1. Verfahren, umfassend:
durch eine Steuerschaltung (101):

Zugreifen auf Informationen bezüglich mindestens eines Sichtfelds eines bestimmten Patienten, wobei das Sichtfeld Informationen bezüglich mindestens eines röntgendichten Objekts enthält;
Verarbeiten der Informationen bezüglich des mindestens einen Sichtfelds unter Verwendung einer Vielzahl von unterschiedlichen Filtern, um eine Vielzahl von gefilterten Sichtfeldern zu erhalten, die sich jeweils voneinander unterscheiden, wobei jedes gefilterte Sichtfeld eine unterschiedlich gefilterte Version des mindestens einen Sichtfelds umfasst; und
**gekennzeichnet durch**:
Fusionieren der Vielzahl von gefilterten Sichtfeldern unter Verwendung einer probabilistischen Fusion mit Mehrfachhinweisen, um fusionierte Informationen bezüglich der Ortung des mindestens einen röntgendichten Objekts durch Unterdrücken unechter Erkennungen zu erhalten.

2. Verfahren nach Anspruch 1, wobei die Informationen bezüglich des mindestens einen Sichtfelds ein Röntgenbild umfassen; und wobei optional die Informationen bezüglich des mindestens einen Sichtfelds nur ein einzelnes Röntgenbild umfassen.

3. Verfahren nach Anspruch 1 oder Anspruch 2, wobei das mindestens eine röntgendichte Objekt eine Bezugsmarke umfasst; und wobei optional die Bezugsmarke eine implantierte Bezugsmarke umfasst.

4. Verfahren nach einem der Ansprüche 1 bis 3, wobei die Vielzahl von unterschiedlichen Filtern mindestens ein Bildfilter umfasst.

5. Verfahren nach Anspruch 4, wobei jedes der Vielzahl von unterschiedlichen Filtern ein Bildfilter umfasst.

6. Verfahren nach Anspruch 4, wobei die Vielzahl von unterschiedlichen Filtern mindestens eines von Folgenden beinhaltet:

ein Konturerkennungsfilter;
ein Bandpassfilter;
ein adaptives Schwellenwertfilter;
ein Bezugsmarkenaufbau-Verbesserungsfilter;
ein maximal stabiles Filter für extreme Regionen;
ein Homogenitätsfilter.

7. Verfahren nach einem der Ansprüche 1 bis 6, wobei die fusionierten Informationen bezüglich des mindestens einen röntgendichten Objekts ein probabilistisch-basiertes Wahrscheinlichkeitsbild umfassen.

8. Verfahren nach Anspruch 7, wobei das probabilistisch-basierte Wahrscheinlichkeitsbild eine wahrscheinlichste Ortung des mindestens einen röntgendichten Objekts identifiziert.

9. Vorrichtung, umfassend:
eine Steuerschaltung (101), die zu Folgendem konfiguriert ist:

Zugreifen auf Informationen bezüglich mindestens eines Sichtfelds eines bestimmten Patienten, wobei das Sichtfeld Informationen bezüglich mindestens eines röntgendichten Objekts enthält;
Verarbeiten der Informationen bezüglich des mindestens einen Sichtfelds unter Verwendung einer Vielzahl von unterschiedlichen Filtern, um eine Vielzahl von gefilterten Sichtfeldern zu erhalten, die sich jeweils voneinander unterscheiden, wobei jedes gefilterte Sichtfeld eine unterschiedlich gefilterte Version des mindestens einen Sichtfelds umfasst; und
**gekennzeichnet durch**:
Fusionieren der Vielzahl von gefilterten Sichtfeldern unter Verwendung einer probabilistischen Fusion mit Mehrfachhinweisen, um fusionierte Informationen bezüglich der Ortung des mindestens einen röntgendichten Objekts durch Unterdrücken unechter Erkennungen zu erhalten.

10. Vorrichtung nach Anspruch 9, wobei die Informationen bezüglich des mindestens einen Sichtfelds ein Röntgenbild umfassen; und wobei optional die Informationen bezüglich des mindestens einen Sichtfelds nur ein einzelnes Röntgenbild umfassen.

11. Vorrichtung nach Anspruch 9 oder Anspruch 10, wobei das mindestens eine röntgendichte Objekt eine Bezugsmarke umfasst; und wobei optional die Bezugsmarke eine implantierte Bezugsmarke umfasst.

12. Vorrichtung nach einem der Ansprüche 9 bis 11, wobei die Vielzahl von unterschiedlichen Filtern mindestens ein Bildfilter umfasst.

13. Vorrichtung nach Anspruch 12, wobei jedes der Vielzahl von unterschiedlichen Filtern ein Bildfilter umfasst.

14. Vorrichtung nach Anspruch 12, wobei die Vielzahl von unterschiedlichen Filtern mindestens eines von Folgenden beinhaltet:

ein Konturerkennungsfilter;
ein Bandpassfilter;
ein adaptives Schwellenwertfilter;
ein Bezugsmarkenaufbau-Verbesserungsfilter;
ein maximal stabiles Filter für extreme Regionen;
ein Homogenitätsfilter.

15. Vorrichtung nach einem der Ansprüche 9 bis 14, wobei die fusionierten Informationen bezüglich des mindestens einen röntgendichten Objekts ein probabilistisch-basiertes Wahrscheinlichkeitsbild umfassen; und wobei optional das probabilistisch-basierte Wahrscheinlichkeitsbild eine wahrscheinlichste Ortung des mindestens einen röntgendichten Objekts identifiziert.

**Revendications**

1. Procédé consistant à :
au moyen d'un circuit de commande (101) :

accéder à des informations concernant au moins un champ de vision d'un patient particulier, lequel champ de vision contient des informations concernant au moins un objet radio-opaque ;
traiter les informations concernant l'au moins un champ de vision à l'aide d'une pluralité de filtres différents pour obtenir une pluralité de champs de vision filtrés qui sont chacun différents les uns des autres, chaque champ de vision filtré comprenant une version filtrée différemment de l'au moins un champ de vision ; et
**caractérisé par** :
la fusion de la pluralité de champs de vision filtrés, en utilisant une fusion probabiliste multi-indices, pour obtenir des informations fusionnées concernant la localisation de l'au moins un objet radio-opaque en supprimant les détections parasites.

2. Procédé selon la revendication 1, dans lequel les informations concernant l'au moins un champ de vision comprennent une image radiologique ; et éventuellement, dans lequel les informations concernant l'au moins un champ de vision ne comprennent qu'une seule image radiologique.

3. Procédé selon la revendication 1 ou la revendication 2, dans lequel l'au moins un objet radio-opaque comprend un repère ; et éventuellement, dans lequel le repère comprend un repère implanté.

4. Procédé selon l'une quelconque des revendications 1 à 3, dans lequel la pluralité de filtres différents comprend au moins un filtre d'image.

5. Procédé selon la revendication 4, dans lequel chacun de la pluralité de filtres différents comprend un filtre d'image.

6. Procédé selon la revendication 4, dans lequel la pluralité de filtres différents comprend au moins l'un parmi :

un filtre de détection de contour ;
un filtre passe-bande ;
un filtre à seuillage adaptatif ;
un filtre d'amélioration de la structure de repère ;
un filtre de régions d'extrema maximalement stable ;
un filtre d'homogénéité.

7. Procédé selon l'une quelconque des revendications 1 à 6, dans lequel les informations fusionnées concernant l'au moins un objet radio-opaque comprennent une image de vraisemblance basée sur des probabilités.

8. Procédé selon la revendication 7, dans lequel l'image de vraisemblance basée sur des probabilités identifie une localisation la plus probable de l'au moins un objet radio-opaque.

9. Appareil comprenant :

un circuit de commande (101) configuré pour :

accéder à des informations concernant au moins un champ de vision d'un patient particulier, lequel champ de vision contient des informations concernant au moins un objet radio-opaque ;

traiter les informations concernant l'au moins un champ de vision à l'aide d'une pluralité de filtres différents pour obtenir une pluralité de champs de vision filtrés qui sont chacun différents les uns des autres, chaque champ de vision filtré comprenant une version filtrée différemment de l'au moins un champ de vision ; et

**caractérisé par** :

la fusion de la pluralité de champs de vision filtrés, en utilisant une fusion probabiliste multi-indices, pour obtenir des informations fusionnées concernant la localisation de l'au moins un objet radio-opaque en supprimant les détections parasites.

10. Appareil selon la revendication 9, dans lequel les informations concernant l'au moins un champ de vision comprennent une image radiologique ; et éventuellement, dans lequel les informations concernant l'au moins un champ de vision ne comprennent qu'une seule image radiologique.

11. Appareil selon la revendication 9 ou la revendication 10, dans lequel l'au moins un objet radio-opaque comprend un repère ; et éventuellement, dans lequel le repère comprend un repère implanté.

12. Appareil selon l'une quelconque des revendications 9 à 11, dans lequel la pluralité de filtres différents comprend au moins un filtre d'image.

13. Appareil selon la revendication 12, dans lequel chacun de la pluralité de filtres différents comprend un filtre d'image.

14. Appareil selon la revendication 12, dans lequel la pluralité de filtres différents comprend au moins l'un parmi :

un filtre de détection de contour ;
un filtre passe-bande ;
un filtre à seuillage adaptatif ;
un filtre d'amélioration de la structure de repère ;
un filtre de régions d'extrema maximalement stable ;
un filtre d'homogénéité.

15. Appareil selon l'une quelconque des revendications 9 à 14, dans lequel les informations fusionnées concernant l'au moins un objet radio-opaque comprennent une image de vraisemblance basée sur des probabilités ; et éventuellement, dans lequel l'image de vraisemblance basée sur des probabilités identifie une localisation la plus probable de l'au moins un objet radio-opaque.

FIG. 1

_200_

By A Control Circuit

_202_

| Field Of View Information Containing Information Regarding At Least One Radiopaque Object | → | Access Information Regarding At Least One Field Of View Of A Particular Patient | ~_201_ |

Process The Information Regarding The At Least One Field Of View Using A Plurality Of Different Filters To Yields A Plurality Of Filtered Fields Of View That Are Each Different From One Another  ~_203_

Fuse The Plurality Of Filtered Fields Of View To Yield Fused Information Regarding The At Least One Radiopaque Object  ~_204_

FIG. 2

FIG. 3

FIG. 4

FIG. 5

500

FIG. 6

_700_

False Positive Rate

Case: Multi-Cue Fusion Auto Beam Hold Symmetrically-Shaped Fiducials

FIG. 7

FIG. 8

_900_

Accuracy

Case: Multi-Cue Fusion Auto Beam Hold Symmetrically-Shaped Fiducials

FIG. 9

1000

FIG. 10

Confidence 0.00    Confidence 0.00    Confidence 0.00

1100

FIG. 11

Confidence 0.00     Confidence 0.00     Confidence 0.00

1200

FIG. 12

Confidence 0.00

M3: Confidence 0.77, Deformation 0.9mm
M3: Confidence 0.93, Deformation 0.3mm
M3: Confidence 0.43, Deformation 0.5mm
M3: Confidence 0.83, Deformation 0.5mm
xxxxxxxxxx
Confidence 0.95

EP 4 494 565 B1

FIG. 13

_1400_

False Positive Rate
Case: Multi-Cue Fusion Auto Beam Hold Symmetrically-Shaped Fiducials

FIG. 14

<u>*1500*</u>

**False Negative Rate**

Case: Multi-Cue Fusion Auto Beam Hold Symmetrically-Shaped Fiducials

FIG. 15

FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017140532 A1

- EP 1760658 B1

**Non-patent literature cited in the description**

- **AHMED IBRAHIM ABDULRAB et al.** Multi-Techniques for Analyzing X-ray Images for Early Detection and Differentiation of Pneumonia and Tuberculosis Based on Hybrid Features. *DIAGNOSTICS*, 20 February 2023, vol. 13, 814